# EUROPÄISCHE PATENTSCHRIFT

(11) **EP 1 015 424 B1**
(45) Veröffentlichungstag und Bekanntmachung des Hinweises auf die Patenterteilung: **17.07.2002**
(21) Anmeldenummer: 98946406.0
(22) Anmeldetag: 21.08.1998
(51) Int. Cl.: C07D 201/08, B01J 21/06

(54) **VERWENDUNG VON FORMKÖRPERN ALS KATALYSATOR ZUR HERSTELLUNG VON CAPROLACTAM**
USE OF SHAPED BODIES AS A CATALYST FOR THE PRODUCTION OF CAPROLACTAM
UTILISATION DE CORPS MOULES COMME CATALYSEUR POUR LA PRODUCTION DE CAPROLACTAME

(30) Priorität: 03.09.1997 DE 19738464
(43) Veröffentlichungstag der Anmeldung: 05.07.2000
(73) Patentinhaber: BASF AKTIENGESELLSCHAFT, 67056 Ludwigshafen (DE)
(72) Erfinder: FUCHS, Eberhard, D-67227 Frankenthal (DE); FLICK, Klemens, D-76863 Herxheim (DE)
(86) Internationale Anmeldenummer: EP9805333
(87) Internationale Veröffentlichungsnummer: WO9911613

(56) Entgegenhaltungen:
- EP-A- 0 551 644
- EP-A- 0 653 381
- EP-A- 0 670 181
- DE-A- 4 339 648
- US-A- 5 484 757

## Beschreibung

Die vorliegende Erfindung betrifft die Verwendung von im wesentlichen ein katalytisch aktives Oxid enthaltenden Formkörpern als Katalysator zur Herstellung von cyclischen Lactamen durch Umsetzung von Aminocarbonsäurenitrilen mit Wasser.

Aus der (OZ.0050/44458) ist die Verwendung von Formkörpern, die unter den Reaktionsbedingungen keine löslichen Bestandteile aufweisen, als Katalysator zur Herstellung cyclischer Lactame durch Umsetzung von Aminocarbonsäurenitrilen mit Wasser in flüssiger Phase in einem Festbettreaktor bekannt. Die Katalysatoren, die eine Vielzahl von Oxiden, Seleniden, Telluriden und Phosphaten umfassen können, sind dabei erhältlich beispielsweise durch Verstrangung von Pulvern der entsprechenden Verbindungen.

Mittels der Formkörper können cyclische Lactame zwar erhalten werden, jedoch sind Selektivität und Ausbeute nicht voll befriedigend insbesondere bei kurzen Verweilzeiten, die eine hohe Raum-Zeit-Ausbeute und somit Verkleinerung der Reaktoren ermöglichen.

Aufgabe der vorliegenden Erfindung war daher die Verwendung von Formkörpern, die unter den Reaktionsbedingungen keine löslichen Bestandteile aufweisen, als Katalysator zur Herstellung cyclischer Lactame durch Umsetzung von Aminocarbonsäurenitrilen mit Wasser in flüssiger Phase in einem Festbettreaktor, die die vorstehend beschriebenen Nachteile nicht mit sich bringen.

Diese Aufgabe wird erfindungsgemäß dadurch gelöst, daß man zur Herstellung cyclischer Lactame durch Umsetzung von Aminocarbonsäurenitrilen mit Wasser in flüssiger Phase in einem Festbettreaktor im wesentlichen ein katalytisch aktives Oxid enthaltende Formkörper als Katalysator, welcher unter den Reaktionsbedingungen keine löslichen Bestandteile aufweist, verwendet, wobei der Katalysator aus Formkörpern besteht, die erhältlich sind durch Formen des Oxids zu Formkörpern und Behandeln des Oxids vor oder nach dem Formen mit 0,1 bis 30 Gew.-% bezogen auf das Oxid einer Säure, in der das Oxid schwerlöslich ist.

Bevorzugte Ausführungsformen der erfindungsgemäßen Verwendung sind den Unteransprüchen zu entnehmen.

Als Ausgangsstoffe der erfindungsgemäßen Verwendung werden Aminocarbonsäurenitrile, vorzugsweise solche der allgemeinen Formel I 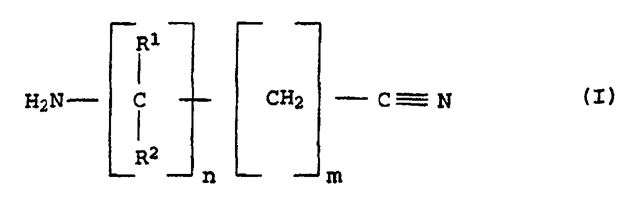 eingesetzt, wobei n und m jeweils die Werte 0, 1, 2, 3, 4, 5, 6, 7, 8 und 9 haben können und die Summe aus n + m mindestens 3, vorzugsweise mindestens 4 beträgt.

R¹ und R² können prinzipiell Substituenten jeglicher Art sein, wobei lediglich sichergestellt sein sollte, daß die gewünschte Cyclisierungsreaktion durch die Substituenten nicht beeinflußt wird. Vorzugsweise sind R¹ und R² unabhängig voneinander C₁-C₆-Alkyl- oder C₅-C₇-Cycloalkylgruppen oder C₆-C₁₂-Arylgruppen.

Besonders bevorzugte Ausgangsverbindungen sind Aminocarbonsäurenitrile der allgemeinen Formel

H₂N―(CH₂)ₘ―C≡N

wobei m einen Wert von 3, 4, 5 oder 6, insbesondere 5 aufweist. Für m = 5 ergibt sich als Ausgangsverbindung 6-Aminocapronsäurenitril.

Erfindungsgemäß werden die vorstehend beschriebenen Aminocarbonsäurenitrile mit Wasser in flüssiger Phase unter Verwendung heterogener Katalysatoren zu cyclischen Lactamen umgesetzt. Bei Verwendung von Aminocarbonsäurenitrilen der Formel I erhält man die entsprechenden cyclischen Lactame der Formel II 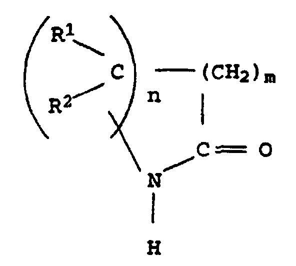 wobei n, m, R¹ und R² die vorstehend genannte Bedeutung haben. Besonders bevorzugte Lactame sind solche, in denen n = O ist und m einen Wert von 4,5 oder 6 hat, insbesondere 5 (im letzteren Fall erhält man Caprolactam).

Die Umsetzung wird in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol Aminocarbonsäurenitril werden im allgemeinen mindestens 0,01 mol, vorzugsweise 0,1 bis 20 und insbesondere 1 bis 5 mol Wasser eingesetzt.

Vorteilhaft wird das Aminocarbonsäurenitril in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Wasser/Lösungsmittel-Gemischen eingesetzt. Als Lösungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, die Aminocarbonsäurenitrile als Reaktand und gleichzeitig Lösungsmittel anzuwenden.

Als katalytisch aktive Oxide können beispielsweise saure, amphotere oder basische Oxide verwendet werden, vorzugsweise Aluminiumoxid, wie alpha- oder gamma-Aluminiumoxid, Zinnoxid, Ceroxid, insbesondere Titandioxid, amorph, als Anatas oder Rutil, sowie deren Gemische und Phasenmischungen.

Die vorstehend genannten Verbindungen können mit Verbindungen der 1. bis 7., insbesondere 2., 3. oder 4. Hauptgruppe des Periodensystems, der 1. bis 7. Nebengruppe des Periodensystems, der Elemente der Eisengruppe oder der Lanthaniden oder Aktiniden sowie Gemischen solcher Verbindungen dotiert sein bzw. diese enthalten. Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Diese katalytisch aktiven Oxide können in sich bekannter Weise, beispielsweise durch Hydrolyse der entsprechenden Organyle, Alkoholate, Salze mit organischen oder anorganischen Säuren und anschließendem Tempern oder Calcinieren sowie vorteilhaft insbesondere im Falle des Titandioxids pyrogen hergestellt werden und sind allgemein kommerziell verfügbar.

Die Oxide werden erfindungsgemäß vor oder nach dem Formen mit einer Säure behandelt. Als Säure kommen organische Säuren wie Essigsäure, Oxalsäure, Propionsäure, Buttersäure, Maleinsäure oder anorganische Säuren wie Isopolysäuren, Heteropolysäuren, Schwefelsäure oder Salzsäure. Besonders geeignete Katalysatoren sind erhältlich durch eine Behandlung mit Essigsäure, Ameisensäure, Salpetersäure, insbesondere Phosphorsäure oder Polyphosphorsäure.

Es können auch Gemische von Säuren eingesetzt werden.

Die Behandlung kann in einer oder in mehreren Stufen kontinierlich oder diskontinuierlich erfolgen, wobei in den einzelnen Stufen die gleiche Säure, verschiedene Säuren oder gleiche oder verschiedene Gemische von Säuren eingesetzt werden können.

Ebenso können die Oxide vor und nach dem Formen in der genannten Art mit einer Säure behandelt werden.

Vorzugsweise werden die Oxide vor dem Formen mit einer Säure behandelt.

Erfindungsgemäß setzt man 0,1 bis 30, vorzugsweise 0,1 bis 10, insbesondere 0,1 bis 5 Gew.-% Säure, berechnet als reine Säure, bezogen auf das pyrogene Titandioxid, ein. Man kann die Säure mit einem flüssigen Verdünnungsmittel, wie Wasser, mischen.

Zur Herstellung der Katalysatoren können die Oxide ohne Zusatzstoffe verwendet werden. Es ist ebenso möglich, Zusatzstoffe, wie Bindemittel, beispielsweise Titandioxid-Sole, Salze der verwendeten Oxide, lösliche Titan-Salz-Verbindungen, hydrolysierbare Titanverbindungen wie Titan-Alkoholate oder Aluminium-Salze, wie Porenbildner, beispielsweise Methylcellulose, Kohlebstoffasern, Fasern organischer Polymere, Melamin, Stärkepulver vorzugsweise vor dem Formen zuzugeben.

Die Formkörper können in verschiedenen Formen vorliegen, beispielsweise als Kugel, Tablette, Zylinder, Hohlzylinder, Pellet, Granulat oder Strang. Derartige Formkörper können in an sich bekannter Weise unter Verwendung zweckentsprechender Formmaschinen wie Tablettiermaschinen, Extrudierformmaschinen, Drehgranulatoren, Pelletisatoren oder Kombinationen solcher Maschinen hergestellt werden.

Das geformte Material wird, gegebenenfalls nach einer Säurebehandlung, vorteilhaft getrocknet, insbesondere bei Temperaturen von 20 bis 120°C, vorzugsweise in einer Inertgasatmosphäre oder an Luft und anschließend calciniert, insbesondere bei 400 - 750°C, vorzugsweise in einer Inertgasatmosphäre oder an Luft.

Die heterogenen Katalysatoren sind in einem Festbett angeordnet. Die Umsetzung kann in an sich bekannter Weise beispielsweise in Riesel- oder vorzugsweise Sumpffahrweise insbesondere kontinuierlich erfolgen, indem das Reaktionsgemisch mit dem Katalysatorbett in Kontakt gebracht wird.

Der Vorteil der erfindungsgemäßen Verwendung liegt in der Möglichkeit, die Cyclisierung auf einfache Weise kontinuierlich zu betreiben bei hohen Ausbeuten und Selektivitäten und kurzen Verweilzeiten mit sehr hohen Durchsätzen. Da die verwendeten Katalysatoren nach bisherigen Beobachtungen eine hohe Lebensdauer aufweisen, ergibt sich ein extrem geringer Katalysator-Verbrauch.

### Beispiel 1: Herstellung von Strängen aus pyrogenem Titatandioxid (Ameisensäure)

8350 g pyrogenes Titandioxid-Pulver mit einem Rutil/Anatas-Verhältnis von 80/20 wurden mit 47 g 85 %iger Ameisensäure und 3750 g Wasser 3 Stunden geknetet und danach in der Strangpresse mit einem Pressdruck von 70 bar zu 4 mm Stängen verformt. Die Stränge wurden für 16 Stunden bei 120°C getrocknet und anschließend für 3 Stunden bei 500°C calziniert.

| Analytik der Stränge: | |
|---|---|
| Litergewicht | 989 g/l |
| Wasseraufnahme | 0,31 ml/g |
| Schneidhärte | 25 N |
| Oberfläche | 37 m²/g |

### Beispiel 2: Herstellung von Strängen aus pyrogenem Titatandioxid (Phosphorsäure)

1950 g gefälltes Titandioxid-Pulver (Anatas) wurden mit 60 g konzentrierter Phosphorsäure und 900 g Wasser 3 Stunden geknetet und danach in der Strangpresse mit einem Pressdruck von 70 bar zu 1,5 mm Strängen verformt. Die Stränge wurden für 6 Stunden bei 120°C getrocknet und anschließend für 5 Stunden bei 350°C calziniert.

| Analytik der Stränge: | |
|---|---|
| Litergewicht | 722 g/l |
| Wasseraufnahme | 0,46 ml/g |
| Oberfläche | 204 m²/g |

### Beispiel 3: Herstellung von Strängen aus gefälltem Titandioxid (Salpetersäure)

11000 g gefälltes Titandioxid-Pulver (Anatas) wurden mit 420 g konzentrierter Salpetersäure und 3650 g Wasser 2 Stunden geknetet und danach in der Strangpresse mit einem Pressdruck von 70 bar zu 3 mm Strängen verformt. Die Stränge wurden für 6 Stunden bei 120°C getrocknet, anschließend für 2 Stunden bei 320°C und für weitere 3 h bei 350°C calziniert.

| Analytik der Stränge: | |
|---|---|
| Litergewicht | 919 g/l |
| Wasseraufnahme | 0,32 ml/g |
| Schneidhärte | 25 N |
| Oberfläche | 105 m²/g |

### Beispiel 4 bis 16: Umsetzung von 6-Aminocapronitril zu Caprolctam

In einen beheizten Rohrreaktor von 25 ml Inhalt (Durchmesser 6 mm; Länge 800 mm), der mit einem aus der Tabelle aufgeführten Katalysatoren 1 bis 4 als Splitt gefüllt war, wurde bei 80 bar eine Lösung von 6-Aminocapronsäurenitril (ACN) in Wasser und Ethanol in den in der Tabelle angegebenen Gewichtsverhältnissen geleitet. Der den Reaktor verlassende Produktstrom wurde gaschromatographisch analysiert. Die Ergebnisse sind in der Tabelle als Beispiele aufgeführt.

Neben Caprolactam enthält der Produktstrom im wesentlichen ε-Aminocapronsäureethylester und ε-Aminocapronsäureamid. Beide lassen sich ebenfalls zu Caprolactam cyclisieren. Zusätzlich findet man 5 bis 8 % Caprolactamoligomere, welche zu Caprolactam gespalten werden können.

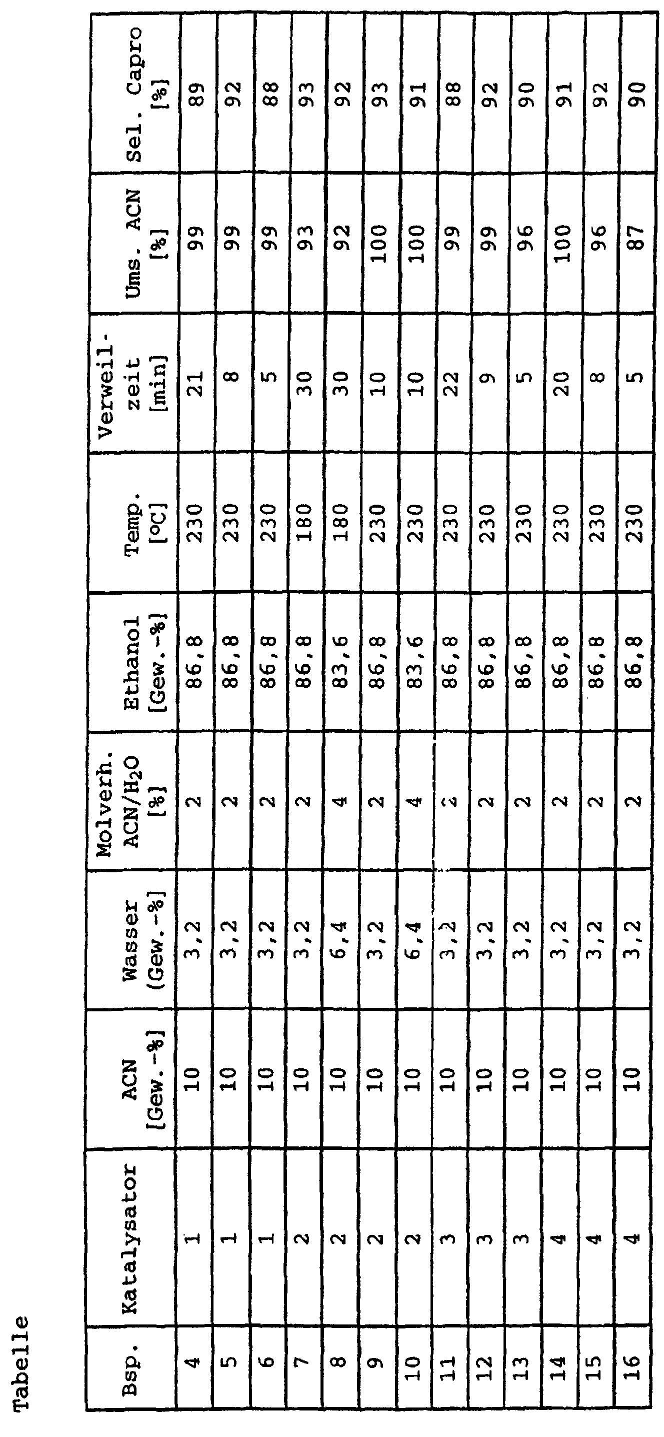

Die Katalysatoren 1 bis 4 sind entsprechend den Katalysatorbeispielen 1 bis 3 hergestellt worden:
- Katalysator 1:: Gefälltes Titandioxid mit 3 % Phosphorsäure zu 3 mm Strängen verstrangt und dann zu 1,0 - 1,5 mm Splitt vermahlen
- Katalysator 2:: Gefälltes Titandioxid mit 3 % Phosphorsäure zu 3 mm Strängen verstrangt
- Katalysator 3:: Pyrogenes Titandioxid mit 3 % Phosphorsäure zu 4 mm Strängen verstrangt und dann zu 1,6 - 2,0 mm Splitt vermahlen
- Katalysator 4:: Pyrogenes Titandioxid mit 0,5 % Ameisensäure zu 4 mm Strängen verstrangt und dann zu 1,6 - 2,0 mm Splitt vermahlen

## Patentansprüche

1. Verwendung von im wesentlichen ein katalytisch aktives Oxid enthaltenden Formkörpern als Katalysator, welcher unter den Reaktionsbedingungen keine löslichen Bestandteile aufweist, zur Herstellung cyclischer Lactame durch Umsetzung von Aminocarbonsäurenitrilen mit Wasser in flüssiger Phase in einem Festbettreaktor, wobei der Katalysator aus Formkörpern besteht, die erhältlich sind durch Formen des Oxids zu Formkörpern und Behandeln des Oxids vor oder nach dem Formen mit 0,1 bis 30 Gew.-% bezogen auf das Oxid einer Säure, in der das Oxid schwerlöslich ist.

2. Verwendung von Formkörpern nach Anspruch 1, **dadurch gekennzeichnet, daß** man die Umsetzung bei einer Temperatur im Bereich von 140 bis 320°C durchführt.

3. Verwendung von Formkörpern nach einem der Ansprüche 1 bis 2, **dadurch gekennzeichnet, daß** man Aminocarbonsäurenitrile der Formel
H₂N―(CH₂)ₘ―C≡N
wobei
m 3, 4, 5 oder 6 ist, einsetzt.

4. Verwendung von Formkörpern nach Anspruch 3, **dadurch gekennzeichnet, daß** man als Aminocarbonsäurenitril 6-Aminocapronsäurenitril einsetzt.

5. Verwendung von Formkörpern nach einem der Ansprüche 1 bis 4, **dadurch gekennzeichnet, daß** man eine 1 bis 50 gew.-%ige Lösung des Aminocarbonsäurenitrils in Wasser oder in Wasser/org. Lösungsmittel-Gemischen einsetzt.

6. Verwendung von Formkörpern nach einem der Ansprüche 1 bis 5, **dadurch gekennzeichnet, daß** der Katalysator als katalytisch aktives Oxid Titandioxid, Aluminiumoxid, Zinnoxid, Zinkoxid, Ceroxid oder deren Gemische enthält.

7. Verwendung von Formkörpern nach einem der Ansprüche 1 bis 6, **dadurch gekennzeichnet, daß** man als Säure Phosphorsäure oder Polyphosphorsäure einsetzt.

8. Verwendung von Formkörpern nach einem der Ansprüche 1 bis 7, **dadurch gekennzeichnet, daß** man als Säure Salpetersäure, Essigsäure oder Ameisensäure einsetzt.

## Claims

1. The use of shaped articles essentially comprising a catalytically active oxide as a catalyst which has no soluble constituents under the reaction conditions for preparing cyclic lactams by reacting aminocarbonitriles with water in the liquid phase in a fixed bed reactor, said catalyst consisting of shaped articles obtainable by shaping the oxide into shaped articles and, before or after said shaping, treating the oxide with from 0.1 to 30% by weight, based on the oxide, of an acid in which the oxide is sparingly soluble.

2. The use of claim 1, **characterized in that** the reaction is carried out at a temperature within the range from 140 to 320°C.

3. The use of either of claims 1 and 2, **characterized in that** the aminocarbonitriles used have the formula
H₂N―(CH₂)ₘ―C≡N
where
m is 3, 4, 5 or 6.

4. The use of claim 3, **characterized in that** the aminocarbonitrile used is 6-aminocapronitrile.

5. The use of any of claims 1 to 4, **characterized in that** the aminocarbonitrile is used in the form of a from 1 to 50% strength by weight solution in water or in water/org. solvent mixtures.

6. The use of any of claims 1 to 5, **characterized in that** the catalytically active oxide is titanium dioxide, aluminum oxide, tin oxide, zinc oxide, cerium oxide or a mixture thereof.

7. The use of any of claims 1 to 6, **characterized in that** the acid used is phosphoric acid or polyphosphoric acid.

8. The use of any of claims 1 to 7, **characterized in that** the acid used is nitric acid, acetic acid or formic acid.

## Revendications

1. Utilisation de corps moulés contenant essentiellement un oxyde à activité catalytique en tant que catalyseur, ne présentant pas de constituants solubles dans les conditions de la réaction, pour la préparation de lactames cycliques par réaction de nitriles aminocarboxyliques avec de l'eau, en phase liquide, dans un réacteur à lit fixe, où le catalyseur consiste en corps moulés que l'on peut obtenir par formage de l'oxyde en corps moulés et traitement de l'oxyde, avant ou après le formage, par 0,1 à 30% en poids, par rapport à l'oxyde, d'un acide, dans lequel l'oxyde est soluble.

2. Utilisation de corps moulés selon la revendication 1, **caractérisée en ce que** l'on entreprend la réaction à une température de l'ordre de 140 à 320°C.

3. Utilisation de corps moulés selon l'une des revendications 1 à 2, **caractérisée en ce que** l'on met en oeuvre des nitriles aminocarboxyliques de la formule
H₂N―(CH₂ₘ―C≡N
dans laquelle
m a une valeur de 3, 4, 5 ou 6.

4. Utilisation de corps moulés selon la revendication 3, **caractérisée en ce que** l'on met en oeuvre du 6-aminocapronitrile en tant que nitrile aminocarboxylique.

5. Utilisation de corps moulés selon l'une des revendications 1 à 4, **caractérisée en ce que** l'on met en oeuvre une solution contenant de 1 à 50% en poids du nitrile aminocarboxylique dans de l'eau ou dans des mélanges d'eau/solvant organique.

6. Utilisation de corps moulés selon l'une des revendications 1 à 5, **caractérisée en ce que** le catalyseur contient en tant qu'oxyde à activité catalytique du dioxyde de titane, de l'oxyde d'aluminium, de l'oxyde d'étain, de l'oxyde de zinc, de l'oxyde de cérium ou leurs mélanges.

7. Utilisation de corps moulés selon l'une des revendications 1 à 6, **caractérisée en ce que** l'on met en oeuvre en tant qu'acides de l'acide phosphorique ou de l'acide polyphosphorique.

8. Utilisation de corps moulés selon l'une des revendications 1 à 7, **caractérisée en ce que** l'on met en oeuvre en tant qu'acides de l'acide nitrique, de l'acide acétique ou de l'acide formique.
